(19) Europäisches Patentamt European Patent Office Office européen des brevets

(11) **EP 4 546 347 A1**

(12) **EUROPEAN PATENT APPLICATION**

(43) Date of publication:
    **30.04.2025 Bulletin 2025/18**

(21) Application number: **24207970.5**

(22) Date of filing: **22.10.2024**

(51) International Patent Classification (IPC):
    **G16B 15/20** (2019.01)    **G16B 15/30** (2019.01)
    **G16B 40/20** (2019.01)

(52) Cooperative Patent Classification (CPC):
    **G16B 15/20; G16B 15/30; G16B 40/20**

(84) Designated Contracting States:
    **AL AT BE BG CH CY CZ DE DK EE ES FI FR GB GR HR HU IE IS IT LI LT LU LV MC ME MK MT NL NO PL PT RO RS SE SI SK SM TR**
    Designated Extension States:
    **BA**
    Designated Validation States:
    **GE KH MA MD TN**

(30) Priority: **23.10.2023 KR 20230142141**

(71) Applicant: **LG Management Development Institute**
    **Seoul 07336 (KR)**

(72) Inventors:
    • **Hwang, Doyeong**
      **07524 Seoul (KR)**
    • **Kim, Kiyoung**
      **07811 Seoul (KR)**
    • **Yim, Soorin**
      **07801 Seoul (KR)**

(74) Representative: **BCKIP Part mbB**
    **MK1**
    **Landsbergerstraße 98, 3.Stock**
    **80339 München (DE)**

(54) **DEVICE FOR PREDICTING PROTEIN INTERACTION OF HLA-PEPTIDE COMPLEX BASED ON ARTIFICIAL INTELLIGENCE AND METHOD USING THE SAME**

(57) A device for predicting protein interaction to predict whether or not protein interact based on artificial intelligence comprises memory; a communication unit; and at least one processor operably connected to the memory and the communication unit. The at least one processor is configured to identify data related to at least one protein sequence from the memory, predict a structure of a first protein complex based on the data related to the at least one protein sequence, determine coordinate information of the first protein complex based on predetermined positional encoding operation, and perform learning to predict interaction between the first protein complex and an external protein based on the coordinate information of the first protein complex.

FIG. 2

EP 4 546 347 A1

## Description

## BACKGROUND

The present disclosure generally relates to a device and method for predicting protein interaction of a human leukocyte antigen(HLA)-peptide complex based on or using artificial intelligence. More specifically, some exemplary embodiments of the present disclosure provide prediction results using positional encoding by utilizing an output learned from protein sequences as input through an artificial intelligence.

A new type of antigen composed of protein complexes may be derived from somatic mutations, and may be formed by the cancer cells and antigen-presenting cells of a subject. Immunogenicity refers to the activation of T cells recognizing a specific antigen. T cells may be activated by recognizing protein complexes in which major histocompatibility complex (MHC) proteins and peptide antigens are bound. For this mechanism, T cell receptors and MHC-peptide protein complexes need to be able to physically bound.

Recently, the performance of various learning models for predicting unknown structures of protein complexes have been developed. However, due to noise or high cost that may occur during the learning process, it may be difficult to directly utilize these models for immunogenicity prediction.

Therefore, a method able to improve the performance of immunogenicity prediction models by utilizing the structure of protein complexes may be needed. Additionally, a learning method that can more accurately predict the physical structure able to bind through the input of given protein sequences may be needed.

## [Related Art Document]

## [Patent Document]

Korean Patent Application Publication No. 10-2022-0064958.

## SUMMARY

An embodiment of the present disclosure may extract a protein complex structure that may be derived from a specific protein sequence based on random protein sequence, thereby providing immunogenicity data corresponding to that specific protein sequence.

According to some embodiments of the present disclosure, a prediction device may provide clear data on residue alpha carbons by searching for a 3D structure of protein complexes likely to be exist through repeated learning.

The problems solved by the present disclosure are not limited to those mentioned above, and other problems not mentioned may be clearly understood by those skilled in the art from the description below.

A device for predicting protein interaction based on artificial intelligence according to an embodiment of the present disclosure may include memory, a communication unit, and at least one processor electrically connected to the memory and the communication unit, wherein the at least one processor is configured to identify data related to at least one protein sequence from the memory, predict the structure of a first protein complex based on the data related to the at least one protein sequence, determine coordinate information of the first protein complex based on predetermined positional encoding operation, and perform learning to predict the interaction between the first protein complex and an external protein based on the coordinate information.

According to an embodiment of the present disclosure, a method for predicting protein interaction to predict whether proteins will interact based on artificial intelligence, performed by at least one processor of a computer device includes identifying data related to at least one protein sequence from memory; predicting the structure of a first protein complex based on the data related to the at least one protein sequence; determining coordinate information of the first protein complex based on predetermined positional encoding operation; and performing learning to predict the interaction between the first protein complex and an external protein based on the coordinate information.

In addition, other method and system for implementing the present disclosure and a computer-readable recording medium storing a computer program for executing the method, may further be provided.

Furthermore, a computer program stored on a medium that allows the method of implementing the certain embodiments of present disclosure to be performed on a computer may further be provided.

According to some embodiments of the present disclosure, a device for predicting protein interaction may extract the structure of a protein complex that may physically bind with a T cell receptor from a protein sequence.

In addition, according to various embodiments of the present disclosure, Fourier feature positional encoding of structural information of protein complexes may be computed and used as input to existing models to improve the performance of immunogenicity prediction models.

The effects of the present disclosure are not limited to those mentioned above, and other effects not mentioned may be clearly understood by those skilled in the art from the description below.

## BRIEF DESCRIPTION OF DRAWINGS

FIG. 1 is a schematic block diagram of a protein interaction prediction device for predicting the structure of a human leukocyte antigen(HLA)-peptide complex according to various embodiments of the present disclosure.

FIG. 2 is a schematic flowchart of a method for predicting the structure of an HLA-peptide complex according to various embodiments of the present disclosure.

FIG. 3 is a flowchart for extracting coordinates of a protein complex according to various embodiments of the present disclosure.

FIG. 4 is a schematic flowchart of a Fourier feature positional encoding process according to various embodiments of the present disclosure.

FIG. 5 is a schematic flowchart of extracting 3D coordinates of a protein complex according to various embodiments of the present disclosure.

FIG. 6 is a flowchart of a method for extracting 3D coordinates through an artificial intelligence learning model according to various embodiments of the present disclosure.

FIG. 7 is a flowchart of a method for determining whether a Fourier feature positional encoding process is applied to a protein complex according to various embodiments of the present disclosure.

FIG. 8 is a flowchart of a method for providing immunogenicity data according to various embodiments of the present disclosure.

## DETAILED DESCRIPTION

Throughout the present disclosure, the same reference numerals designate the same components. The present disclosure does not describe all elements of the embodiments, and general contents in the technical field of the present disclosure or duplicated content among the embodiments are omitted. The terms "unit, module, member, block" used in the specification may be implemented in software or hardware, and depending on the embodiments, multiple "units, modules, members, blocks" may be implemented as a single component, or a single "unit, module, member, block" may also include multiple components.

Throughout the specification, when a part is described as being "connected" to another part, it includes not only cases where they are directly connected but also cases where they are indirectly connected, which may be connected by a wireless communication network.

Furthermore, when a part is described as "comprising" a certain component, it means that it may further include other components, not excluding other components unless explicitly stated otherwise.

Throughout the specification, when one member is described as being "on" other member, it includes not only cases where the members are in contact but also cases where another member exists between them.

Terms such as "first" and "second" are used to distinguish one component from another, and are not intended to limit the components to those aforementioned by the terms.

Singular expressions include plural expressions unless the context clearly indicates otherwise.

Identification codes used for each step are provided for convenience in description and do not specify the order of the steps, and each step may be carried out in a different order unless a specific order is explicitly described.

The operating principles and embodiments of the present disclosure will be described below with reference to the accompanying drawings.

The term "device according to the present disclosure" in this specification encompasses various devices capable of performing operations and providing results to users. For example, the device according to the present disclosure may include a computer, server device, and portable terminal, or it may take any one of these forms.

Herein, the computer may include, for example, a notebook, desktop, laptop, tablet personal computer (PC), or slate PC, equipped with a web browser.

The server device is a server that processes information by communicating with an external device, and may include an application server, a computing server, a database server, a file server, a game server, a mail server, a proxy server, and a web server.

The portable terminal is for example, a wireless communication device ensuring portability and mobility, and may include all kinds of handheld-based wireless communication devices such as Personal Communication System (PCS), Global System for Mobile communications (GSM), Personal Digital Cellular (PDC), Personal Handyphone System (PHS), Personal Digital Assistant (PDA), International Mobile Telecommunication (IMT)-2000, Code Division Multiple Access (CDMA)-2000, W-Code Division Multiple Access (W-CDMA), Wireless Broadband Internet (WiBro) terminal, smart phone, and wearable devices such as watches, rings, bracelets, anklets, necklaces, glasses, contact lenses, or head-mounted devices (HMD).

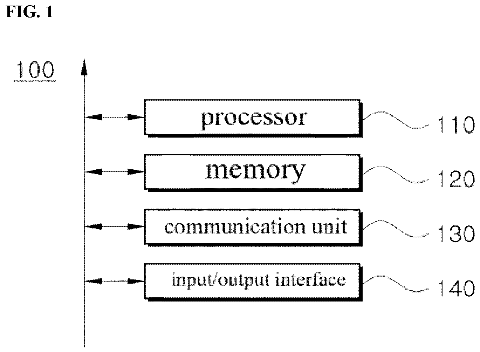

FIG. 1 is a schematic block diagram of a protein interaction prediction device for predicting the structure of a human leukocyte antigen(HLA)-peptide complex according to various embodiments of the present disclosure.

A device 100 for predicting protein interaction may include one or more processors 110, memory 120, a communicator or communication unit 130, and an input and output interface 140, and the like. However, internal components that may be included in the device 100 for predicting protein interaction are not limited to this exemplary embodiment of the present disclosure. For instance, the device 100 for predicting protein interaction may perform processing functions of a processor through a separate processing server or a cloud server instead of including the processor 110 in the device 100.

Referring to FIG. 1, the processor 110 may be configured to perform one or more operations of the device 100 for predicting protein interaction in association with the memory 120 that stores data on an algorithm for controlling the operations of components included in or associated with the device 100 for predicting protein interaction or a program that reproduces the algorithm, and the data stored in the memory 120. For example, the processor 110 and memory 120 may be implemented as separate chips. Alternatively, the processor 110 and the memory 120 may be implemented as a single integrated chip.

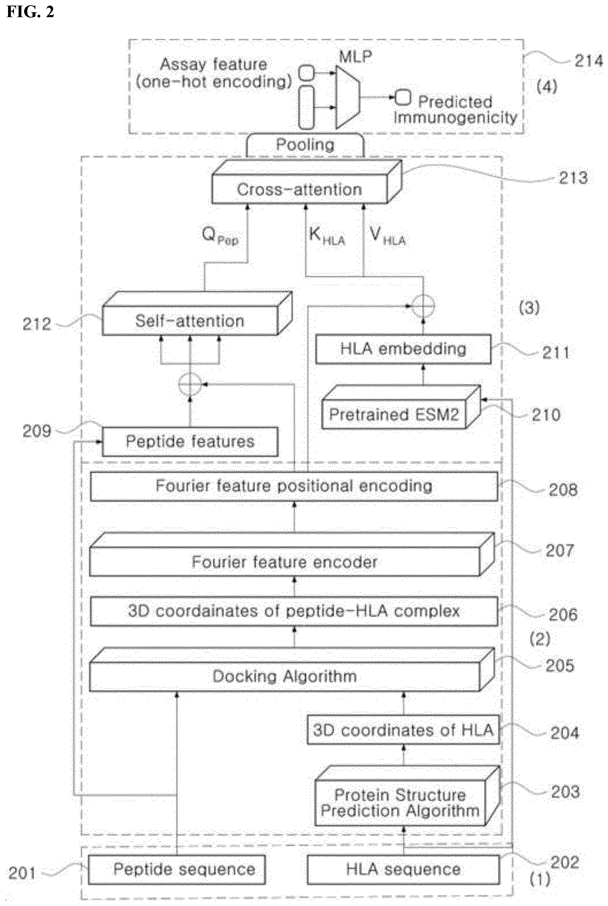

The processor 110 may control one or more of the components, in the device 100 for predicting protein interaction, described above to implement various embodiments of the present disclosure as described in FIGS. 2 to 7.

The memory 120 according to an embodiment may store data performing or supporting various functions of the device 100 for predicting protein interaction, programs for the operations of the processor 110, input and/or output data (e.g., images, videos, etc.), multiple application programs or applications running on the device for predicting protein interaction 100, and data or instructions for operating the device for predicting protein interaction 100. At least some of these application programs may be downloaded from an external server via wired or wireless communication.

The memory 120 may include at least one type of storage medium, such as flash memory type, hard disk type, Solid State Disk (SSD) type, Silicon Disk Drive (SDD) type, multimedia card micro type, card type memory (e.g., SD or XD memory), random access memory (RAM), static random access memory (SRAM), read-only memory (ROM), electrically erasable programmable read-only memory (EEPROM), programmable read-only memory (PROM), magnetic memory, magnetic disk, and optical disk. Additionally, the memory may be a database that is separate from the device for predicting protein interaction 100, but is connected wired or wirelessly.

The communicator or communication unit 130 according to an embodiment may include one or more components configured to communicate with external devices, including, for example, at least one of a broadcast receiving module or broadcast receiver, wired communication module or wired communicator, wireless communication module or wireless communicator, short-range communication module or short-range communicator, or location information module.

The wired communication module may include various wired communication modules such as a Local Area Network (LAN) module, a Wide Area Network (WAN) module, or a Value Added Network (VAN) module, as well as various cable communication modules such as Universal Serial Bus (USB), High Definition Multimedia Interface (HDMI), Digital Visual Interface (DVI), recommended standard 232 (RS-232), power line communication, or plain old telephone service (POTS).

The wireless communication module may include not only a WiFi module, a Wireless broadband (WiBro) module, but also a wireless communication module supporting various wireless communication methods such as Global System for Mobile Communication (GSM), Code Division Multiple Access (CDMA), Wideband Code Division Multiple Access (WCDMA), Universal Mobile Telecommunications System (UMTS), Time Division Multiple Access (TDMA), Long Term Evolution (LTE), 4G (Generation), 5G, and 6G.

The short-range communication module is for short-range communication and may support short-range communication using at least one of the following technologies: Bluetooth™, Radio Frequency Identification (RFID), Infrared Data Association (IrDA), Ultra Wideband (UWB), ZigBee, Near Field Communication (NFC), Wireless-Fidelity (Wi-Fi), Wi-Fi Direct, or Wireless Universal Serial Bus (Wireless USB).

The input and/or output interface 140 according to an embodiment serves as a channel for connecting various types of external devices to the device 100 for predicting protein interaction. The input and/or output interface 140 may include, for example, but not limited to, at least one of the following: a wired and/or wireless headset port, an external charger port, a wired and/or wireless data port, a memory card port, a port for connecting a device equipped with a Subscriber Identification Module (SIM), an audio Input/Output (I/O) port, a video Input/Output (I/O) port, or an earphone port. The device 100 for predicting protein interaction 100 may perform control related to the external device connected to the input and/or output interface 140.

One or more modules or components may be added or removed to or from the device 100 illustrated in FIG. 1 if necessary. 1. It will be readily understood by those skilled in the art that the mutual positions of the components may also be modified in accordance with the performance or structure of the device 100.

Meanwhile, each module or component shown in FIG. 1 represents software and/or hardware components such as a Field Programmable Gate Array (FPGA) and an Application Specific Integrated Circuit (ASIC).

FIG. 2 is a schematic flowchart of a method for predicting the structure of an HLA-peptide complex according to various embodiments of the present disclosure.

Referring to FIG. 2, a peptide sequence 201 may be one of protein sequences and may be a sequence representing a subset of a protein in which amino acids are connected in a single row. The peptide sequence may be represented as a string indicating the order of amino acids connected by peptide bonds. Human leukocyte antigen (HLA) sequence 202 may refer to a sequence of HLA molecules corresponding to proteins that play an role in the human immune system. Herein, HLA is a protein complex that is used to represent antigens recognized mainly by immune cells such as T cells and B cells. The HLA sequence represents the amino acid sequence of HLA molecules, which may be experimentally determined using sequencing technology or searched through online resources.

A Protein Structure Prediction Algorithm 203 according to an embodiment may be a deep learning-based model developed for predicting the 3D structure of protein complexes. The Protein Structure Prediction Algorithm 203 may predict the 3D structure from the one-dimensional amino acid sequence of a protein utilizing a technique called Markov Deep Learning. For example, the Protein Structure Prediction Algorithm 203 may include an algorithm that utilizes the ESMfold model. The 3D coordinates 204 of the HLA may be 3D coordinates of the HLA predicted by the Protein Structure Prediction Algorithm 203 based on the HLA sequence 201.

A Docking Algorithm 205 according to an embodiment may be an algorithm that utilizes an operational model to predict structural changes of a protein alone or a protein-ligand complex. The Docking Algorithm 205 may predict the structural changes occurring in the ligand-bound protein structure and the effects of ligand binding. For example, the Docking Algorithm 205 may include an algorithm that utilizes a DiffDock model. The Docking Algorithm 205 may be configured for structural prediction of protein complexes, protein-ligand interaction analysis, drug design, and the like.

3D coordinates 206 of the HLA-peptide complex 206 according to an embodiment may be extracted from the 3D coordinates of the HLA-peptide complexes extracted via the second model based on the 3D coordinate of the HLA and the peptide sequence. The Fourier feature encoder 207 is an encoder configured to convert input data into a low-dimensional feature space via Fourier feature mapping. The Fourier feature encoder 207 may be an encoder of machine learning models that primarily process image or spatial data.

According to an embodiment, Fourier feature positional encoding 208 may be one of encoding schemes used in natural language processing (NLP) models. For example, Fourier feature positional encoding 208 may be primarily used in a transformer model. A peptide feature 209 may refer to a property or characteristic of the data used to describe the characteristics of the peptide. Peptide features 209 may include values that numerically represent the physical, chemical, and/or structural properties of a peptide sequence.

The pre-learned ESM2 210 according to an embodiment may, for example, but not limited to, be a second version of an evolutionary scale modeling (ESM) model which is previously learned. The ESM model may be a deep learning model that is utilized to understand and handle protein sequences. The ESM model may be used to predict the structure of protein complexes, inter-protein interactions, protein-ligand interactions, and the like.

Referring to FIG. 2, HLA embedding 211 may be a technique used to effectively represent HLA molecules that play an important role in the human immune system. HLA may have unique HLA types for each individual, as each genotype has a variety of alleles. HLA embedding 211 may be a technique that represents an HLA type as a high-dimensional vector. HLA embedding 211 can more easily represent similarities and differences between HLA types, with features corresponding to a specific HLA type. An embedding vector may have hundreds or more dimensions and may represent an HLA with a position on a multidimensional space instead of a single number.

According to an embodiment, self-attention 212 may be one of components of a transformer, which is a deep learning model used in the field of natural language processing (NLP). Self-attention 212 may be configured to calculate a correlation of each position with another position for every position in a given input sequence. As a result, individual words (e.g., tokens) of the input sequence may be represented by vectors, and self-attention 212 may calculate relevance how much individual word vectors are associated with other word vectors. Cross attention 213 is configured to model the interaction with other input sequences.

An array feature according to an embodiment may refer to a characteristic expressed in a multidimensional arrangement form in data. MLP is a multilayer perceptron and may be a type of artificial neural network. The MLP is a deep learning model having multiple hidden layers, and there may be multiple neurons in the individual hidden layers 214.

According to an embodiment, a processor (e.g., the processor 110 in FIG. 1) may identify data related to at least one protein sequence from memory (e.g., the memory 120 in FIG. 1). For example, at least one protein sequence may comprise an HLA sequence and a peptide sequence. The processor may determine, based on the HLA sequence and the peptide sequence, whether the T cell has a structure of a protein complex that may be recognized and activated as a specific antigen.

According to an embodiment, the processor may predict a first protein complex based on data relating to at least one protein sequence. For example, the protein complex may comprise an HLA-peptide complex. That is, the processor may provide immunogenicity data through data learned about the structure of whether a T cell is a protein complex that may be recognized and activated as a particular antigen.

According to an embodiment, the processor may extract the 3D coordinates of the HLA through the first model based on the HLA sequence. For example, the first model may be an ESMfold (Evolutionary Scale Modeling) model. The processor may insert the HLA sequence as an input value into the first model, and predict a 3D coordinate (e.g., a 3D structure) of the HLA with respect to the input value. That is, the processor may extract the 3D coordinates of the HLA through the learning model based on the HLA sequence.

According to an embodiment, the processor may extract 3D coordinates of the HLA-peptide complex through a second model based on the 3D coordinates and the peptide sequence of the HLA. For example, the second model may be a DiffDock model. The processor may insert the 3D coordinates of the HLA and the peptide sequence as input values into the second model, and predict the 3D coordinate (e.g., 3D structure) of the HLA-peptide complex with respect to the input value. That is, the processor may extract the 3D coordinates of the HLA-peptide complex through a learning model based on the peptide sequence and the 3D coordinate of the HLA extracted from the HLA sequence.

According to an embodiment, the processor may calculate Fourier feature positional encoding from the 3D coordinates of the first protein complex based on a predetermined operation. The Fourier feature positional encoding may be one of encoding schemes used in natural language processing (NLP) models. For example, the Fourier feature positional encoding may be primarily used in a transformer model. The transformer model is a model for embedding individual tokens of an input sequence, and then adding a positional embedding to inform the model of the word order of the sentence. However, since the transformer model may not have the word position information of the sequence, it may be a force to learn the relative position of the input token. The Fourier feature positional encoding uses a Fourier transform to encode the frequency information of the sequence, thereby adding periodic signals to the individual positions of the input sequence to convey the relative positional information of the words to compensate for the drawbacks of the transformer model. Accordingly, the Fourier feature positional encoding may inform the model of the order of words by adding periodic patterns to each position of the input sentence. This allows the transformer model to utilize the relative positional information of the words to make it easier to understand and process the structure of the characters. The processor extracts 3D coordinates of the protein complex from the protein complex using the Fourier feature positional encoding, and performs a Fourier feature positional encoding operation from the extracted 3D coordinates. For this operation, the processor may utilize a Fourier feature encoder model. The processor may calculate Fourier feature positional encoding using the 3D structure for each residue of the HLA-peptide complex as an input value. For example, Fourier feature positional encoding may be performed by the following equations:

[Equation 1]

$$F \leftarrow \frac{1}{\sqrt{|F|}}[\cos XW_r^T ; \sin XW_r^T]$$

[Equation 2]

$$Y \leftarrow \mathrm{GeLU}(FW_1 + B_1)W_2 + B_2$$

[Equation 3]

$$PE_X \leftarrow \mathrm{Reshape}\ Y\ \text{into the shape of}\ [N, D]$$

In the above Equations 1 to 3, $X \in R^{N \times |F|}$ may refer to the 3D coordinate data x of an individual residue alpha carbon (M = 3). According to an embodiment, the processor may calculate Fourier feature positional embedding to extract PEx. $W_r \in R^{\frac{|F|}{2} \times M}$, $W_1 \in R^{|F| \times |H|}$, $W_2 \in R^{|H| \times |D|}$ represents learnable weights, while $B_1 \in R^{|H|}$ and $B_2 \in R^{|D|}$ may represent learnable biases. Additionally, $|F|$, $|H|$, $|D|$ represent input feature, hidden, and dimension of output, respectively, N represents the sequence length, and " " represents the concatenation operation.

According to the embodiment, the processor may extract the 3D coordinates of the alpha carbon from the 3D coordinate of the HLA-peptide complex. The processor may then be configured to calculate the positional encoding from the 3D coordinates of the individual residue alpha carbon.

According to an embodiment, the processor may identify the 3D coordinates of the protein complex being pre-stored from the memory. The 3D coordinates of the pre-stored protein complex may be already known coordinates. This is because if the coordinates are already known, there is no need to identify the coordinates (structure) again. Here, the 3D coordinates of the pre-stored protein complex will be described as the 3D coordinate of second protein complex. The processor determines whether the first protein complex and the second protein complex to be aware of the immunogenicity prediction data have a matching structure. This operation is for confirming whether a structure in which the processor is pre-stored and a structure to be newly searched match each other. The processor may perform operations of calculating positional encoding from the 3D coordinates of the second protein complex if the first protein complex and the second protein complex match each other. However, if the first protein complex and the second protein complex do not match each other, an overall process of searching for immunogenicity data for a new protein complex (e.g., an HLA-peptide complex) may be performed, for example, as in the operations in FIG. 2.

According to an embodiment, the processor may perform self-attention by summing the peptide feature extracted based on the peptide sequence and the positional encoding. The processor may extract an embedded HLA embedding through a pre-learned model based on the HLA sequence. Here, the pre-learned model may be, for example, but not limited to, ESM2. The processor may perform cross-attention with a result of summing the positional encoding and the HLA embedding together with a result of performing self-attention. In this case, the processor may apply an individual weight to the 3D coordinates of the individual residue alpha carbon in the positional encoding operation.

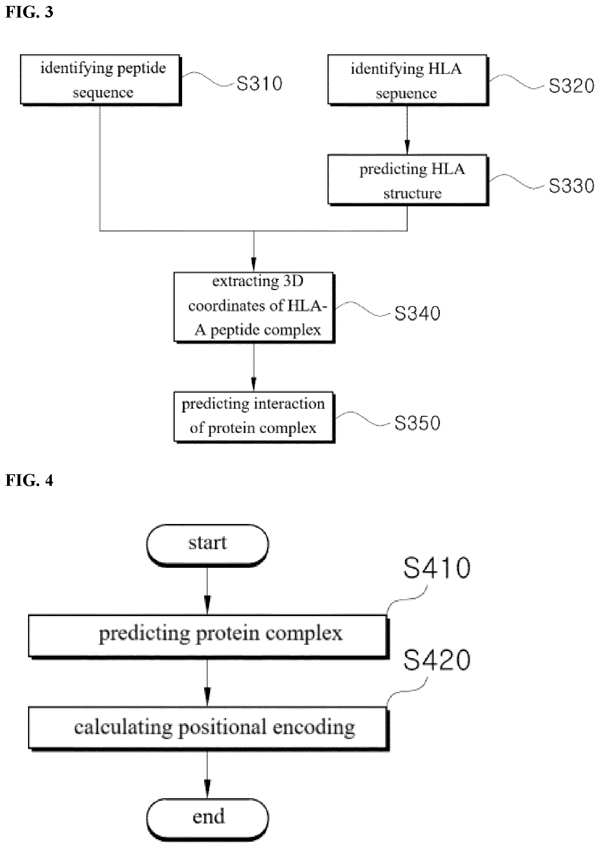

FIG. 3 is a flowchart of extracting coordinates of a protein complex according to various embodiments of the present disclosure. Referring to FIG. 3, in step S310, a processor (e.g., the processor 110 of FIG. 1) may identify a peptide sequence. The processor may extract a sequence of a given peptide and may identify the HLA sequence in parallel in step S320.

In step S330, the processor may predict the HLA structure. For example, the processor may utilize ESMfold to predict the 3D structure of the HLA from the HLA sequence. This may correspond to extracting the 3D coordinates of the HLA.

In step S340, the processor may predict the structure of the HLA-peptide complex. The processor may predict the structure of the HLA peptide complex by utilizing DiffDock from the peptide sequence identified in step S310 and the 3D coordinates of the HLA extracted in step S330.

In step S350, the processor may extract 3D coordinates of the protein complex. The processor may then predict the interaction of the protein complex via the predetermined Fourier feature positional encoding in step S360.

FIG. 4 is a schematic flowchart of a Fourier feature positional encoding process according to various embodiments of the present disclosure.

At step S410, a processor (e.g., the processor 110 of FIG. 1) may predict first protein complex based on data regarding at least one protein sequence. Herein, the at least one protein sequence may comprise an HLA sequence and a peptide sequence. The processor obtains a result of inputting the HLA sequence through a first model. The processor may then predict the first protein complex via second model using the obtained result and the peptide sequence as inputs. The prediction of the first protein complex may comprise 3D coordinates of the first protein complexes.

In step S420, the processor may calculate Fourier feature positional encoding. The processor computes the Fourier feature positional encoding from the 3D coordinates of the first protein complex via a Fourier feature encoder model. Particularly, the processor extracts the 3D coordinates of the individual residue alpha carbon from the 3D coordinate of the HLA-peptide complex. The processor may calculate the Fourier feature positional encoding from the 3D coordinates of the individual residue alpha carbon. Accordingly, the processor can provide the user with immunogenicity data corresponding to the first protein complex.

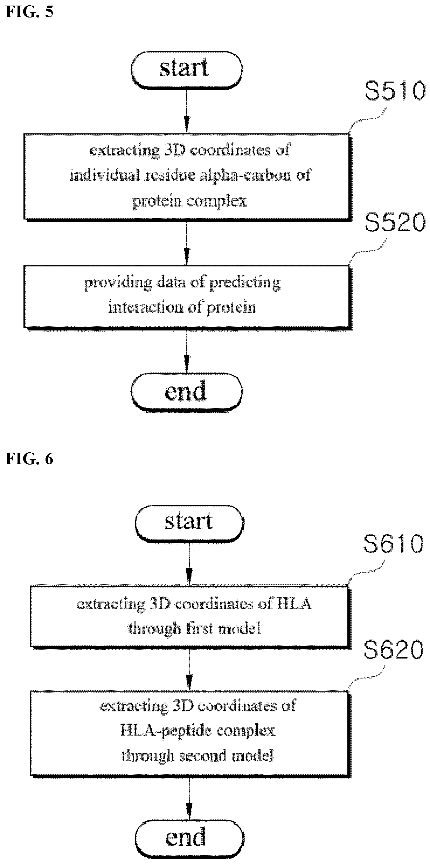

FIG. 5 is a schematic flowchart of 3D coordinate extraction of a protein complex according to various embodiments of the present disclosure. Referring to FIG. 5, in step S510, a processor (e.g., the processor 110 of FIG. 1) may extract the 3D coordinates of individual residue alpha carbon positioned at the 3D coordinate of the first protein complex.

In step S520, the processor may provide the user with protein interaction prediction data corresponding to the first protein complex and the external protein. For example, the processor may use the 3D coordinates of the extracted HLA-peptide complex as an input data set to repeatedly extract which HLA-peptide complexes interact with external proteins. The processor may store the extracted result in the memory to provide the predicted result as interaction data according to which HLA-peptide complex is given based on the cumulatively learned result. Specifically, the processor may extract the 3D coordinates of the HLA obtained by learning the HLA sequence through the first model and the 3D coordinate of the HLA-peptide complex obtained by learning through the second model using the 3D coordinate and the peptide sequence of the HLA as input data in the individual layers. More specifically, the processor may extract the 3D coordinates of the peptide sequence and the HLA-peptide complex obtained through the HLA sequence to predict what interaction the complex is expected to have with an external protein.

FIG. 6 is a flowchart of a method for extracting 3D coordinates through an artificial intelligence learning model according to various embodiments of the present disclosure.

Referring to FIG. 6, in S610, a processor (e.g., the processor 110 in FIG. 1) may extract 3D coordinates of the HLA through a first model. The first model may be an ESMfold model. The processor may input the HLA sequence as an input value into the first model, and predict 3D coordinates (e.g., a 3D structure) of the HLA with respect to the input value. That is, the processor may extract the 3D coordinates of the HLA through the learning model based on the HLA sequence.

In step S620, the processor may extract the 3D coordinates of the HLA-peptide complex through a second model based on the 3D coordinate of the HLA and the peptide sequence. For example, the second model may be a DiffDock model. The processor may input the 3D coordinates of the HLA and the peptide sequence as input values into the second model, and predict the 3D coordinate (e.g., 3D structure) of the HLA-peptide complex with respect to the input values. That is, the processor may extract the 3D coordinates of the HLA-peptide complex through a learning model based on the peptide sequence and the 3D coordinate of the HLA extracted from the HLA sequence. Because the peptide size of the immunogenic data is not large, the DiffDock model may be able to utilize peptides expressed in fine-grained form as input.

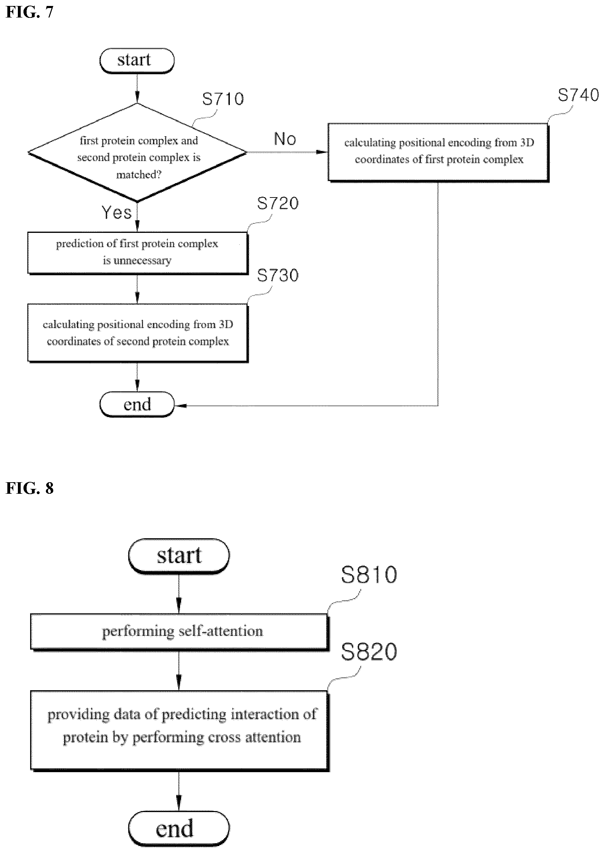

FIG. 7 is a flowchart of a method for determining whether a Fourier feature positional encoding process is applied to a protein complex according to various embodiments of the present disclosure.

Referring to FIG. 7, in step S710, a processor (e.g., the processor 110 in FIG. 1) may identify the 3D coordinates of the protein complex pre-stored in memory (e.g., the memory 120 in FIG. 1). The 3D coordinates of the pre-stored protein complex may be already known coordinates. This is because if the coordinates are already known, there is no need to confirm the coordinates (e.g. a structure) again. This may be to confirm whether a structure in which the processor is pre-stored and a structure to be newly searched match. The processor determines whether the first protein complex and the second protein complex to know the immunogenicity prediction data have a structure matching each other.

If it is determined that the first protein complex and the second protein complex do not match each other at step S710, the processor may determine that prediction of the first protein complex does not need to be performed (step S720). The processor may then proceed to step S730 to calculate Fourier feature positional encoding from the 3D coordinates of the second protein complex. This is a possible process because it is already known coordinates.

If it is determined that the first protein complex and the second protein complex match each other at step S710, the processor may calculate positional encoding from the 3D coordinates of the first protein complex (step S740). If the first protein complex and the second protein complex do not match each other, an overall process of searching for immunogenicity data for a new protein complex (e.g., an HLA-peptide complex) may be performed, for example, as in the operation in FIG. 2 of the present disclosure.

FIG. 8 is a flowchart of a method for providing immunogenicity data according to various embodiments of the present disclosure. In step S810, a processor (for example, the processor 110 in FIG. 1) may sum the peptide feature extracted based on the peptide sequence and the positional encoding to perform self-attention. The processor may extract an embedded HLA embedding through a pre-learned model based on the HLA sequence. Here, the pre-learned model may be, for example, but not limited to, ESM2. In step S820, the processor may perform cross-attention on the result of summing the positional encoding and the HLA embedding together with the result of performing self-attention. As a result, the processor may provide protein interaction prediction data corresponding to the first protein complex and the external protein by performing cross attention.

Meanwhile, certain disclosed embodiments may be implemented in the form of a recording medium that stores instructions executable by a computer. The instructions may be stored in the form of program code and, when executed by a processor, may generate program modules to perform the operations of the disclosed embodiments. The recording medium may be implemented as a computer-readable recording medium.

The computer-readable recording medium includes any type of recording medium in which instructions that may be decrypted by a computer are stored. For example, Examples include a read only memory (ROM), a random access memory (RAM), a magnetic tape, a magnetic disk, a flash memory, an optical data storage device, and the like.

As described above, the disclosed embodiments are described with reference to the accompanying figures. A person of ordinary skill in the art may understand that the present disclosure may be implemented in a different form from the disclosed embodiments without changing the technical sprit or essential features of the present disclosure. The disclosed embodiments are illustrative and restrictive.

**[Explanation of Symbols]**

100: device for predicting protein interaction

110: processor

120: memory

130: communication unit

140: input/output interface

**Claims**

1. A system comprising:

memory; and
at least one processor operably connected to the memory,
wherein the at least one processor is configured to:

identify data related to at least one protein sequence from the memory,
predict a structure of a first protein complex based on the identified data related to the at least one protein sequence,
determine coordinate information of the first protein complex based on a predetermined positional encoding operation, and
perform learning to predict interaction between the first protein complex and an external protein based on the coordinate information of the first protein complex.

2. The system of claim 1, wherein:

the at least one protein sequence comprises a human leukocyte antigen (HLA) sequence and a peptide sequence,
the first protein complex comprises an HLA-peptide complex,
the coordinate information of the first protein complex includes 3D coordinates of the first protein complex, and
the at least one processor is configured to:

calculate positional encoding from the 3D coordinates of the first protein complex based on the predetermined positional encoding operation, and
provide immunogenicity data corresponding to the first protein complex by performing cross-attention on a peptide feature extracted based on the at least one protein sequence, HLA embedding, and the calculated positional encoding.

3. The system of claim 2, wherein:
the at least one processor is configured to extract 3D coordinates of the HLA through a first model based on the HLA sequence.

4. The system of claim 3, wherein:
the at least one processor is configured to extract 3D coordinates of the HLA-peptide complex through a second model based on the 3D coordinates of the HLA and the peptide sequence.

5. The system of claim 4, wherein:
the at least one processor is configured to:

extract 3D coordinates of individual residue alpha carbons from the 3D coordinates of the HLA-peptide complex, and
perform the predetermined positional encoding operation from the 3D coordinates of the individual residue alpha carbons, and
the predetermined positional encoding is Fourier feature positional encoding.

6. The system of claim 4, wherein:
the at least one processor is configured to:

identify 3D coordinates of a second protein complex from the memory, and
determine whether a structure of the first protein complex and a structure of the second protein complex match each other.

7. The system of claim 6, wherein:
the at least one processor is configured to perform the predetermined positional encoding operation from the 3D coordinates of the second protein complex if the structure of the first protein complex and the structure of the second protein complex match each other.

8. The system of claim 2, wherein:
the at least one processor is configured to:

perform self-attention by summing the peptide feature and an output acquired by performing the predetermined positional encoding operation,
extract an embedded HLA embedding through a pre-learned model based on the HLA sequence, and
perform the cross-attention on a result of summing the calculated positional encoding, the extracted HLA embedding, and an output acquired by performing the self-attention.

9. The system of claim 5, wherein:
the at least one processor is configured to, when performing the predetermined positional encoding operation, apply individual weights to the 3D coordinates of the individual residue alpha carbons.

10. A computer-implemented method comprising:

identifying data related to at least one protein sequence from memory;
predicting a structure of a first protein complex based on the identified data related to the at least one protein sequence;
determining coordinate information of the first protein complex based on a predetermined positional encoding operation; and
performing learning to predict interaction between the first protein complex and an external protein based on the coordinate information of the first protein complex.

11. The computer-implemented method of claim 10, wherein:

the at least one protein sequence comprises a human leukocyte antigen (HLA) sequence and a peptide sequence,
the first protein complex comprises an HLA-peptide complex,
the coordinate information of the first protein complex includes 3D coordinates of the first protein complex, and
the at least one processor calculates positional encoding from the 3D coordinates of the first protein complex based on the predetermined positional encoding operation, and provides immunogenicity data corresponding to the first protein complex by performing cross-attention on a peptide feature extracted based on the at least one protein sequence, HLA embedding, and the calculated positional encoding.

12. The computer-implemented method of claim 11, wherein:
the at least one processor extracts 3D coordinates of the HLA through a first model based on the HLA sequence.

13. The computer-implemented method of claim 12, wherein:
the at least one processor extracts 3D coordinates of the HLA-peptide complex through a second model based on the 3D coordinates of the HLA and the peptide sequence.

14. The computer-implemented method of claim 13, wherein the at least one processor:

extracts 3D coordinates of individual residue alpha carbons from the 3D coordinates of the HLA-peptide complex, and
performs the predetermined positional encoding operation from the 3D coordinates of the individual residue alpha carbons, and
wherein the predetermined positional encoding is Fourier feature positional encoding.

15. The computer-implemented method of claim 13, wherein the at least one processor:

identifies 3D coordinates of a second protein complex from the memory, and
determines whether a structure of the first protein complex and a structure of the second protein complex match each other.

16. The computer-implemented method of claim 15, wherein the predetermined positional encoding operation from the 3D coordinates of the second protein complex is performed if the structure of the first protein complex and the structure of the second protein complex match each other.

17. A non-transitory computer-readable storage medium having instructions that, when executed by one or more processors, cause the one or more processors to:

identify data related to at least one protein sequence from memory;
predict a structure of a first protein complex based on the identified data related to the at least one protein sequence;
determine coordinate information of the first protein complex based on a predetermined positional encoding operation; and
perform learning to predict interaction between the first protein complex and an external protein based on the coordinate information of the first protein complex.

**FIG. 1**

100

| | |
|---|---|
| processor | 110 |
| memory | 120 |
| communication unit | 130 |
| input/output interface | 140 |

**FIG. 2**

**FIG. 3**

```
┌──────────────────┐                    ┌──────────────────┐
│ identifying peptide │──── S310        │ identifying HLA   │──── S320
│     sequence      │                   │    sepuence       │
└──────────────────┘                    └──────────────────┘
         │                                        │
         │                              ┌──────────────────┐
         │                              │ predicting HLA    │──── S330
         │                              │   structure       │
         │                              └──────────────────┘
         │                                        │
         │            ┌──────────────────┐        │
         └───────────▶│ extracting 3D     │◀───────┘
                      │ coordinates of HLA-│──── S340
                      │  A peptide complex │
                      └──────────────────┘
                               │
                      ┌──────────────────┐
                      │ predicting interaction│──── S350
                      │  of protein complex │
                      └──────────────────┘
```

**FIG. 4**

```
              ┌──────────┐
              │  start    │
              └──────────┘
                   │
    ┌─────────────────────────────────┐
    │   predicting protein complex     │──── S410
    └─────────────────────────────────┘
                   │
    ┌─────────────────────────────────┐
    │  calculating positional encoding  │──── S420
    └─────────────────────────────────┘
                   │
              ┌──────────┐
              │   end     │
              └──────────┘
```

**FIG. 5**

```
            ┌─────────┐
            │  start  │
            └─────────┘
                 │
                 ▼
   ┌──────────────────────────────┐        S510
   │    extracting 3D coordinates of │
   │ individual residue alpha-carbon of │
   │         protein complex        │
   └──────────────────────────────┘
                 │
                 ▼
   ┌──────────────────────────────┐        S520
   │   providing data of predicting  │
   │     interaction of protein      │
   └──────────────────────────────┘
                 │
                 ▼
            ┌─────────┐
            │   end   │
            └─────────┘
```

**FIG. 6**

```
            ┌─────────┐
            │  start  │
            └─────────┘
                 │
                 ▼
   ┌──────────────────────────────┐        S610
   │    extracting 3D coordinates of HLA │
   │         through first model     │
   └──────────────────────────────┘
                 │
                 ▼
   ┌──────────────────────────────┐        S620
   │    extracting 3D coordinates of │
   │       HLA-peptide complex       │
   │        through second model     │
   └──────────────────────────────┘
                 │
                 ▼
            ┌─────────┐
            │   end   │
            └─────────┘
```

**FIG. 7**

```
          start
            │
            ▼                         S710
        ┌─────────┐
       ╱ first protein ╲      No      ┌──────────────────────────┐  S740
      ╱ complex and     ╲─────────────▶│ calculating positional   │
      ╲ second protein  ╱              │ encoding from 3D          │
       ╲ complex is    ╱               │ coordinates of first      │
        ╲ matched?    ╱                │ protein complex           │
         └─────────┘                   └──────────────────────────┘
         Yes │                                      │
             ▼              S720                     │
   ┌──────────────────────┐                          │
   │ prediction of first  │                          │
   │ protein complex is   │                          │
   │ unnecessary          │                          │
   └──────────────────────┘  S730                     │
             │                                        │
             ▼                                        │
   ┌──────────────────────┐                           │
   │ calculating positional│                          │
   │ encoding from 3D      │                          │
   │ coordinates of second │                          │
   │ protein complex       │                          │
   └──────────────────────┘                           │
             │                                         │
             ▼                                         │
           end ◀─────────────────────────────────────┘
```

**FIG. 8**

```
          start
            │
            ▼                      S810
   ┌──────────────────────────┐
   │ performing self-attention │
   └──────────────────────────┘  S820
            │
            ▼
   ┌──────────────────────────────────┐
   │ providing data of predicting      │
   │ interaction of protein by         │
   │ performing cross attention        │
   └──────────────────────────────────┘
            │
            ▼
           end
```

15

Europäisches
Patentamt
European
Patent Office
Office européen
des brevets

# EUROPEAN SEARCH REPORT

Application Number

EP 24 20 7970

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AKBAR RAHMAD ET AL: "Progress and challenges for the machine learning-based design of fit-for-purpose monoclonal antibodies", MABS, vol. 14, no. 1, 16 March 2022 (2022-03-16), XP055920363, US ISSN: 1942-0862, DOI: 10.1080/19420862.2021.2008790 Retrieved from the Internet: URL:https://www.ncbi.nlm.nih.gov/pmc/articles/PMC8928824/pdf/KMAB_14_2008790.pdf> * the whole document * | 1-17 | INV. G16B15/20 G16B15/30 G16B40/20 |
| X | CN 116 206 675 B (BEIJING FENZIZHIXIN TECH CO LTD) 15 September 2023 (2023-09-15) * abstract * * paragraph [0033] - paragraph [0045] * * figures 1-3 * | 1-17 | |
| A | Evans Richard ET AL: "Protein complex prediction with AlphaFold-Multimer", bioRxiv, 10 March 2022 (2022-03-10), XP093020077, DOI: 10.1101/2021.10.04.463034 Retrieved from the Internet: URL:https://www.biorxiv.org/content/10.1101/2021.10.04.463034v2.full.pdf [retrieved on 2023-02-02] * the whole document * | 1-17 | TECHNICAL FIELDS SEARCHED (IPC) G16B |

-/--

The present search report has been drawn up for all claims

| Place of search | Date of completion of the search | Examiner |
|---|---|---|
| Munich | 19 March 2025 | Schechner-Resom, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

**EP 4 546 347 A1**

| | Europäisches Patentamt European Patent Office Office européen des brevets | **EUROPEAN SEARCH REPORT** | Application Number EP 24 20 7970 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JISNA V A ET AL: "Protein Structure Prediction: Conventional and Deep Learning Perspectives", PROTEIN JOURNAL, KLUWER ACADEMIC/PLENUM PUBLISHERS, DORDRECHT, NL, vol. 40, no. 4, 28 May 2021 (2021-05-28), pages 522-544, XP037542047, ISSN: 1572-3887, DOI: 10.1007/S10930-021-10003-Y [retrieved on 2021-05-28] * abstract * * page 530 - page 541 * | 1-17 | |
| A | BOZHEN HU ET AL: "Protein Language Models and Structure Prediction: Connection and Progression", ARXIV.ORG, CORNELL UNIVERSITY LIBRARY, 201 OLIN LIBRARY CORNELL UNIVERSITY ITHACA, NY 14853, 30 November 2022 (2022-11-30), XP091382005, * the whole document * | 1-17 | |
| A | XIAO CHEN ET AL: "DProQ: A Gated-Graph Transformer for Protein Complex Structure Assessment", ARXIV.ORG, CORNELL UNIVERSITY LIBRARY, 201 OLIN LIBRARY CORNELL UNIVERSITY ITHACA, NY 14853, 21 May 2022 (2022-05-21), XP091229914, * abstract * | 1-17 | TECHNICAL FIELDS SEARCHED (IPC) |

The present search report has been drawn up for all claims

| Place of search | Date of completion of the search | Examiner |
|---|---|---|
| Munich | 19 March 2025 | Schechner-Resom, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 24 20 7970

This annex lists the patent family members relating to the patent documents cited in the above-mentioned European search report.
The members are as contained in the European Patent Office EDP file on
The European Patent Office is in no way liable for these particulars which are merely given for the purpose of information.

19-03-2025

| Patent document cited in search report | Publication date | Patent family member(s) | Publication date |
|---|---|---|---|
| CN 116206675 B | 15-09-2023 | CN 116206675 A | 02-06-2023 |
| | | CN 117292743 A | 26-12-2023 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220064958